# EUROPEAN PATENT SPECIFICATION

(11) **EP 1 331 885 B1**
(45) Date of publication and mention of the grant of the patent: **11.03.2009**
(21) Application number: 01980098.6
(22) Date of filing: 07.11.2001
(51) Int. Cl.: A61B 17/00

(54) **A DEVICE FOR PLUGGING AN OPENING SUCH AS IN A WALL OF A HOLLOW OR TUBULAR ORGAN**
VORRICHTUNG ZUM ABDICHTEN EINER ÖFFNUNG BEISPIELSWEISE IN EINER WAND EINES HOHLEN ODER ROHRFÖRMIGEN ORGANS
DISPOSITIF D'OCCLUSION D'UNE OUVERTURE TELLE QUE DANS UNE PAROI D'ORGANE CREUX OU TUBULAIRE

(30) Priority: 07.11.2000 US 707691
(43) Date of publication of application: 06.08.2003
(60) Divisional application: 08012442.3
(73) Proprietor: Carag AG, 6340 Baar (CH)
(72) Inventor: SOLYMAR, Laszlo, S-427 36 Billdal (SE)
(74) Representative: Clerc, Natalia
(86) International application number: PCT/CH2001/000653
(87) International publication number: WO 2002/038051

(56) References cited:
- WO-A-97/41779
- US-A- 3 874 388
- US-A- 4 836 204
- US-A- 5 171 259
- US-A- 5 976 174
- US-A- 6 117 159

## Description

### FIELD OF THE INVENTION

The present invention generally relates to an implant device for closing a body passage (e.g., an aperture through the auricle diaphragm or the ventricle diaphragm of a heart) or in a body channel, and more particularly comprising an occluding or closing body which is expanded and fixed into place at the passage area to be occluded/closed.

### BACKGROUND OF THE INVENTION

By way of example EP B1 0 362 113 shows a device for closing a passage in the heart of patients, wherein the closing part runs the risk of tipping over and thereby causing the passage to be exposed in passing through the heart. The cause of the non-secure closing capacity is the relative unfixed maneuverability for the closing part when applied around the passage, and that the application takes part long before it has arrived in its final position around the passage in the heart.

WO 97/41779 discloses a device for plugging an opening in a wall of a hollow or tubular organ according to the preamble of claim 1.

The main object of the present invention is therefore primarily to try to solve the described problem with maneuverability and application of the device.

### SUMMARY OF THE INVENTION

The foregoing object is achieved by means of a device according to the present invention, which is substantially **characterized in that** a fluid tight closing body that expands and stiffens in a radial direction is arranged to be built up or otherwise assembled at the position of the intended closing spot, after insertion through a body vein.

The present invention provides an implant according to claim 1 for occluding a passage in a circulatory system.

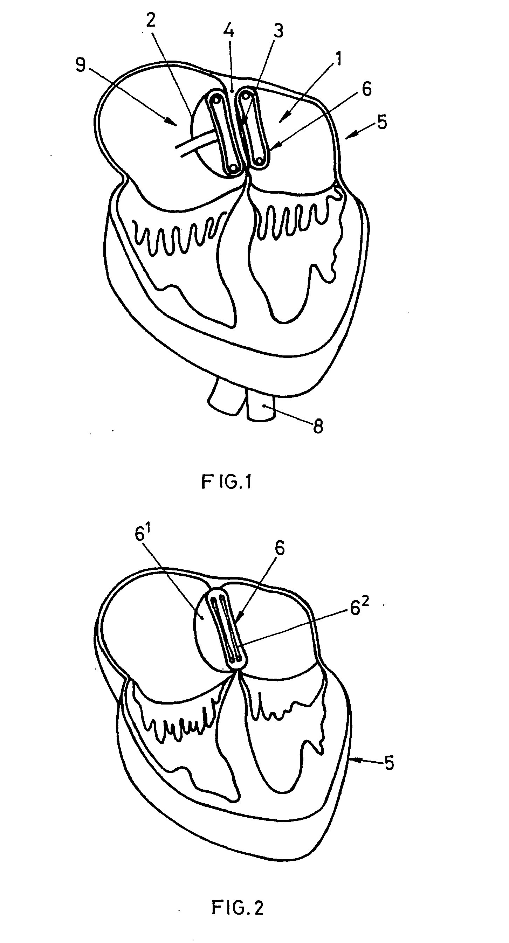

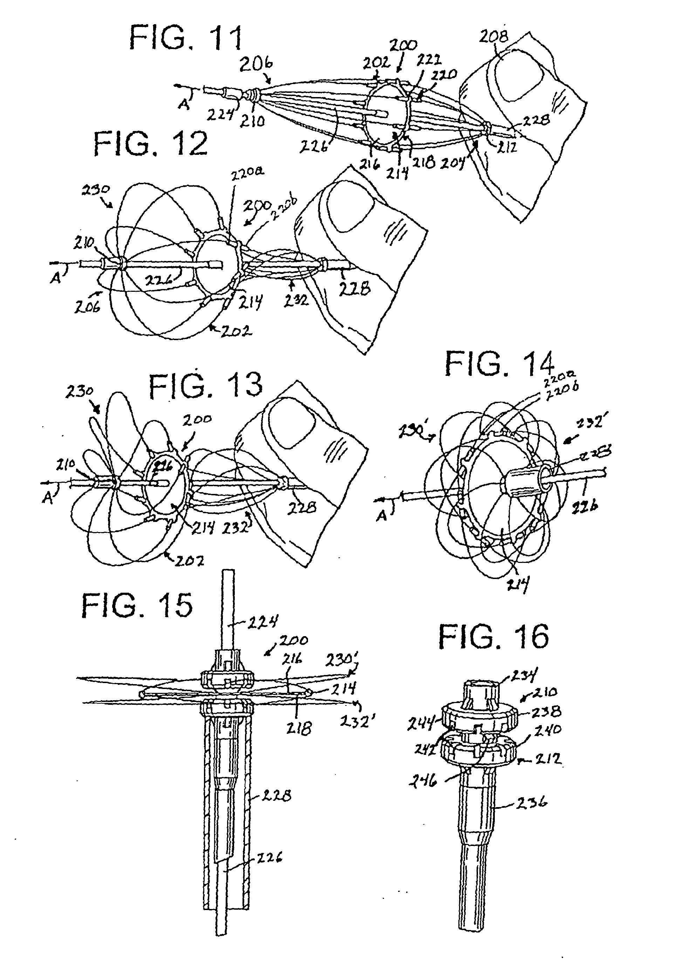

These and other advantages and objects achieved by the present invention will be further appreciated and understood upon consideration of the following detailed description of certain embodiments taken in conjunction with the drawings in which only Figs. 11-16 show a device according to the invention as defined in claim 1, whereas the devices shown in Figs. 1-10 do not fall under the scope of the claims.

### BRIEF DESCRIPTION OF THE DRAWINGS

**FIG.1** shows a section through a heart during application of the implant in it;
**FIG. 2** shows the implant positioned in the heart;
**FIGS. 3-5** relate to a first embodiment of an implant, wherein

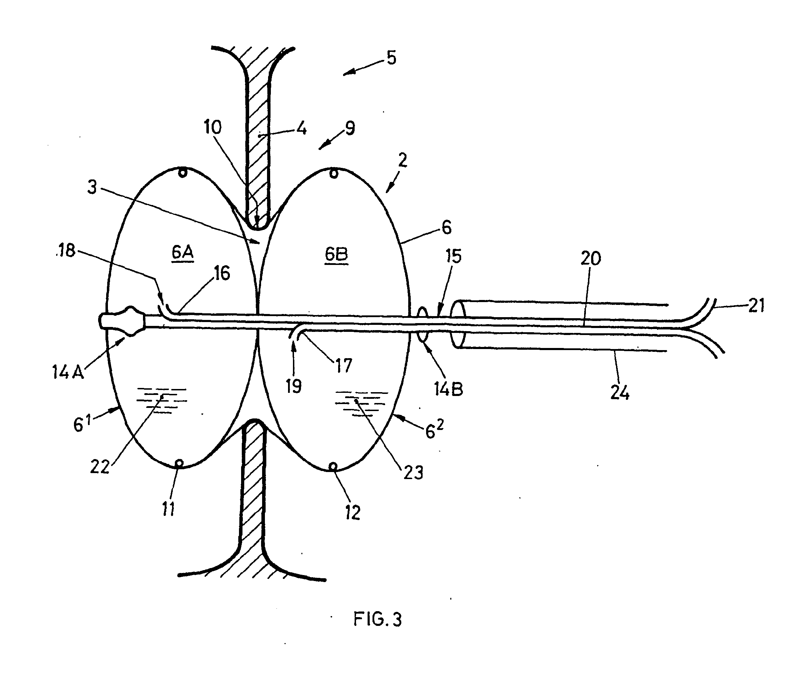

**FIG. 3** shows the implant moved into position before fixing;

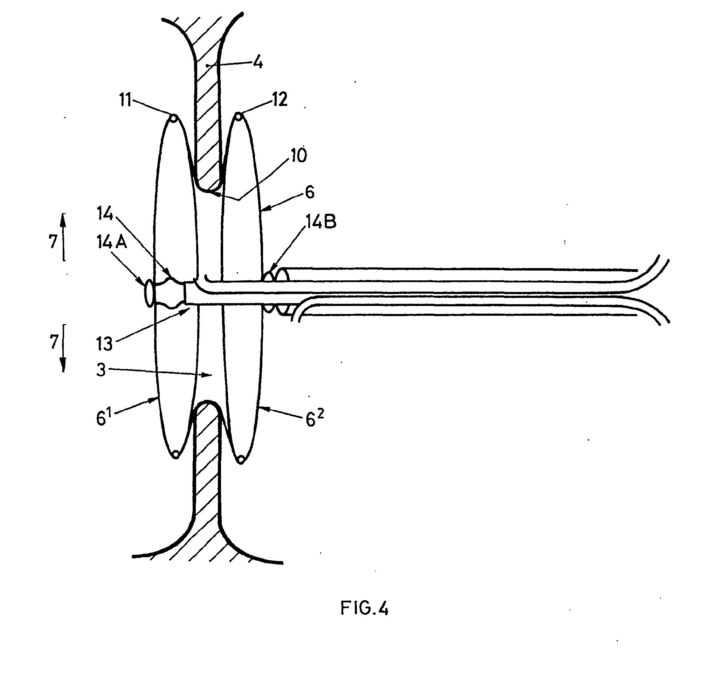

**FIG. 4** shows the implant below a fixing position;

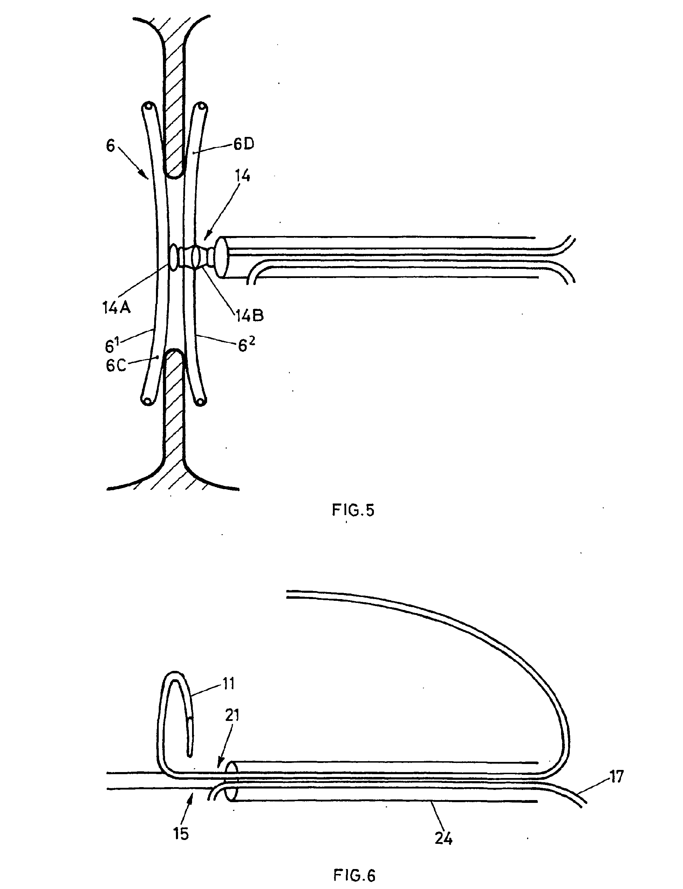

**FIG. 5** shows the implant in its fixing position;
**FIG. 6** shows application of a spiral thread via sheaths;

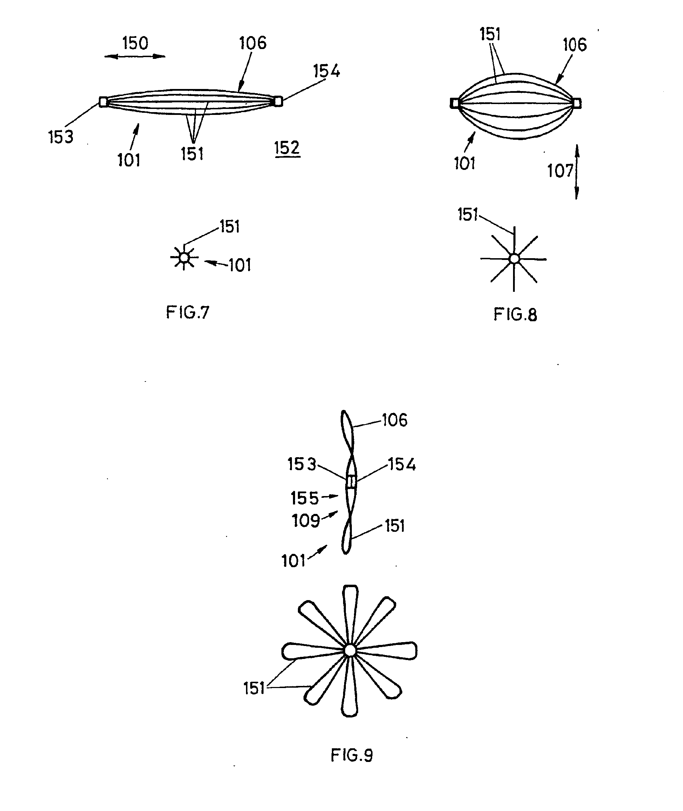

**FIGS. 7-16** relate to a second embodiment of an implant, wherein
**FIG. 7** shows an initial position for the implant seen from the side and from one end respectively;

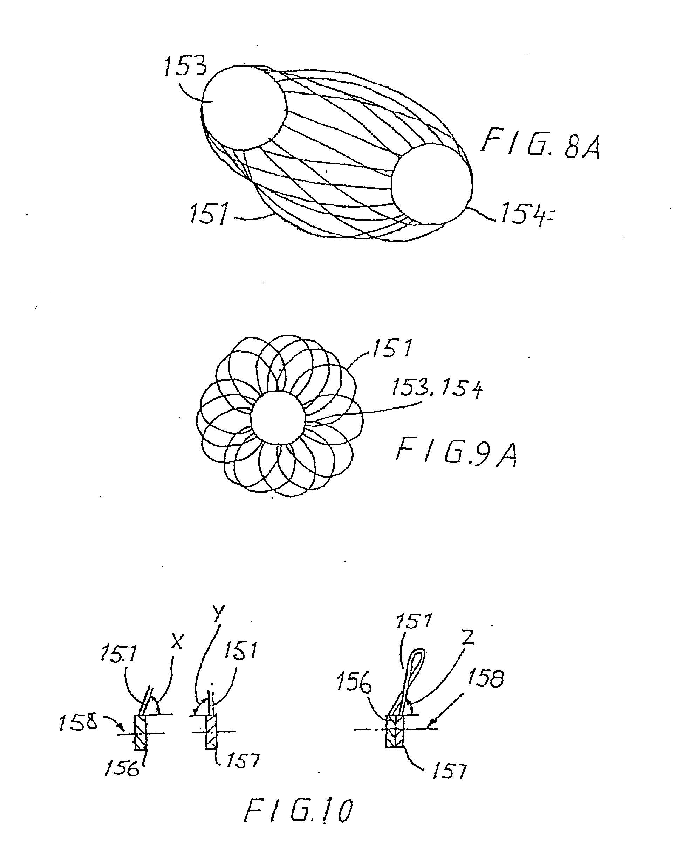

**FIG. 8** shows the implant at a between position during extension seen from the side and one end respectively;
**FIG. 9** shows the implant completely extended seen from the side and one end respectively;
**FIG. 8A** shows the implant in a perspective view during extension;
**FIG. 9A** shows an end view of the implant in its extended position;
**FIG. 10** shows schematically the principle for building up the hub and connection of the implant to it and the final position of the implant in completely extended condition seen from the side;
**FIG. 11** shows the second embodiment of the present invention in another perspective view in an initial position;
**FIG. 12** shows the implant of **FIG. 11** in a perspective view in a first partially deployed position;
**FIG. 13** shows the implant of **FIG. 11** in a perspective view in a second partially deployed position;
**FIG. 14** shows the implant of **FIG. 11** in a perspective view in a fully deployed condition;
**FIG. 15** shows the implant of **FIG. 11** in a side view in a fully deployed condition; and
**FIG. 16** shows an enlarged perspective view of a portion of the implant, without the membrane and loops, detailing the locking hubs.

### DETAILED DESCRIPTION OF EMBODIMENTS OF THE INVENTION

The invention has found particular application as a cardiological implant by means of which it is possible, for example, to close an aperture through the auricle diaphragm or the ventricle diaphragm of a heart.

The implant according to the present invention is arranged to be deployed or built up (i.e., assembled) at a desired location in the body (e.g., the heart), in contrast to known implants (e.g., so-called umbrellas and sails) that are instead extended as soon as the compressed umbrella leaves its insertion sheath.

According to the invention, a device 1 is employed as an implant 2 for closing an internal passage 3, for example, an aperture in the auricle diaphragm 4 or the ventricle diaphragm of a heart 5, or in a desired body channel which one wishes to close. A closing body 6 is deployed or built up at the location. More particularly, an embodiment of the invention that is shown on the drawings in **FIGS. 1-5****,** is formed in a radial direction 7 of expanding and stiffening fluid tight closing body 6. This body 6 is arranged to be built up at the position 9 of the intended closing spot, after insertion through a body vein 8.

The closing body 6 in this first embodiment consists of an inflatable balloon, preferably a double balloon of thin and non-thrombogenic material. A connection part 10 between the two balloon elements 6A, 6B is arranged to form a guidance for the two balloon elements 6A, 6B around the peripheral edge 3A of aperture 3.

Both chambers 6A, 6B of the balloon 6 are arranged to be dilated (expanded) radially 7 by means of a number of stiffening means 11, 12 which in the first shown example is realized in the shape of a coil spring, which in each case is received in the respective balloon chamber 6¹, 6², in order to dilate the balloon chamber 6¹, 6² radially in the operational position and thereby bring about an efficient holding of the balloon around the aperture 3, as is shown in **FIG. 4****.**

Centrally at the middle 13 of the balloon is located a locking mechanism 14, which is arranged to mutually interconnect both balloon chambers 6¹, 6² at their central part. Suitably, the aforementioned locking mechanism 14 is divided into two parts, with each respective locking member 14A, 14B attachable to the outer wall 6C, 6D of the respective balloon chamber 6¹, 6². For example, the aforementioned locking members 14A, 14B may be of a prior art type, e.g., as snap together members.

The described implant 2 is delivered to the location for application in the form of a tightly rolled up double balloon 6 of thin, durable and physically friendly non-thrombogenic material over an inner sheath 15. Two narrow delivery catheters 16, 17 extend through the inner sheath 15, of which the distal delivery catheter 16 has its outlet opening 18 in the distal balloon 6¹ and in a corresponding manner, the proximal catheter 17 has its outlet opening 19 in the proximal balloon 6².

By means of the well-known Seldinger technique, a vein inserter of dimension 11F is inserted into the femoral vein. A catheter is placed in the left upper pleural vein, and through this is passed a conductor 20 which is left behind for subsequent implant work. At the same time, this constitutes a part of a safety system against unintentional release, because the conductor 20 locks the fastening mechanism between the inner sheath 15 and the implant 2. Over the conductor 20, the implant 2 is introduced into the vein inserter, then in the lower vena cava and further up to the heart 5 until the central mark reaches the middle of the aperture 3, or any other defect which is desired to be closed. After that, the distal balloon 6¹ is filled with contrast fluid via the distal delivery catheter 16, whereupon the intended metal spiral 11 is inserted into the delivery catheter 21 and is fed in until it rolls itself up inside the distal balloon 6¹. In a corresponding way, the proximal balloon 6² is filled with contrast fluid in order to enable the parts to be visible via roentgen or other imaging means inside the body during the work to move them into position. After that a metal spiral 12 is also delivered to the proximal balloon 6², as is shown in **FIG. 3****.** When the metal spirals 11, 12, or like stiffening functioning means are located in the correct position, they are released by backing the constraining mandrins out of the metal spirals 11, 12. The contrast fluid 22, 23 is evacuated out from the balloons 6¹, 6² and the distal part 14 of the locking mechanism is pulled through its proximal ring 14B in the locking position. The conductor 20 is then pulled out accompanied by the inner sheath 15 which now runs freely and is pulled out from the distal balloon attachment and also the outer enclosing delivery sheath 24.

The embodiment which is shown in **FIG. 7-10** departs from the above described first embodiment, foremost by instead of using metal spirals as dispersing force which maintains a balloon or other occluding sail in position, it instead utilizes the rotating quality of non-bendable metals when they are compressed, and when the ends move past a critical point. For this object, there are thin nitinol threads attached around an inner core of material in each end inside a balloon according to the above. The distal and the proximal balloons have a further opening between them and the diameter of the balloon at its smallest part corresponding to the ASD-size, i.e. that it is somewhat but not much larger. The balloons are inflated by means of a contrast fluid whereupon the furthermost, as seen in the direction of insertion, attachment point of the nitinol threads are slowly pulled back at the same time as the nearest, as seen in the direction of insertion, are pushed ahead, i.e. in the direction toward each other. At a critical position, the threads twist or spiral sideways and maintain a circular shape, which desired shape is used according to the present invention. In position, the balloons are evacuated and the threads are locked in the twisted spring-like position.

More precisely, with reference to **FIGS. 7-10** a device **101** is formed as an implant for closing a passage, for example, according to the above described embodiment, and which comprises a closing body 106 which is attachable at the passage, alone or in combination with further sealing means in a radial direction 107 which is also an expanding stiffening fluid tight closing body. The body 106 is arranged that after insertion through a body vein (not shown) in its longitudinal direction 150, it will be built up at the position 109 for the intended closing spot.

A number of threads 151 which in the shown embodiment are eight in number, are arranged of such a material and with such qualities that they twist automatically sideways to form a circular or loop shape when compressed longitudinally, as is shown in **FIG. 9****,** and are locked in the twisted, spiral or coil-like position for interconnecting the two chambers, and so on, against each other. The body 106 is preferably formed by a plurality of thin nitinol threads, or wires, which are attached respectively in axial direction 150, 152 relative to each other on movable holder nuclei 153, 154. The threads 151 are arranged to twist sideways in the same direction and then to assume a somewhat circular shape 155, similar to a flower, a propeller or an umbrella, with circular shape and arranged with suitable means for closing the passage. For example, the closing portion may be formed of layers of watertight material connected to the threads 151.

In the example according to **FIG. 10** the threads 151 are shown attached with different inclination x, y with reference to the respective separate holder nuclei 156, 157. The threads 151 are arranged to be mutually forced to be directed toward that holder nucleus 157 from which the threads 151 depart with the largest angle y, as calculated from the center axis 158 of the holder nuclei, with similar angle z for the different threads 151.

**FIGS. 11-16** further illustrate the foregoing embodiment of the present invention. **FIG. 11** shows the implant 200 of the present invention in a perspective view in an initial position, i.e., before the device is applied to the passage in a circulatory system and deployed. In the figures a plurality of thin stiff generally inextensible, but somewhat flexible, members or wires 202 are provided. The members 202 are preferably nitinol. While the figure shows eight members, it should be understood that the number of members can be varied and not change the spirit of the invention.

Each member 202 has a proximal end 204 and a distal end 206. The proximal end 204 is shown here positioned adjacent the user's hand 208 while the distal end 206 enters the passage (not shown) first In practice, of course, the hand would typically be much further away, given that the device will be introduced intravenously. The distal ends 206 of the inextensible members 202 are attached to a first holder 210 that has a ring shape like that of a circular hub or hub flange. The proximal ends 204 of the inextensible members 202 are attached to a second holder 212 similar to the first holder 210, i.e., a ring-shaped holder or hub.

At a point generally midway between the first holder 210 and the second holder 212 an expansible occluding body 214 is attached to the members 202. The occluding body 214 may be a generally circular, disc-shaped member, and may be made of a flexible fabric-like material consistent with surgical use. The occluding body 214 has a distal face 216 oriented toward the distal ends 206 of the members 202 and a proximal face 218 oriented toward the proximal ends 204 of the members 202. The members 202 preferably have a thickened portion 220 where they pass through openings 222 in the occluding body 214. The thickened portion 220 is actually two parts 220a, 220b which serve to capture and mount the occluding body 214 therebetween.

The first holder 210 is attached to a first end 224 of a carrier rod 226 at the distal ends 206 of the members 202. The second holder 212 is slidably received on the carrier rod 226. In the condition shown in **FIG. 11****,** the members 202 are arranged about the; carrier rod 226 in a substantially equally spaced manner and generally aligned with the longitudinal axis A of the carrier rod 226. The occluding body 214 extends in a generally radial manner from the carrier rod 226 at a mid-point between the proximal 204 and distal ends 206 of the inextensible members. In the arrangement shown in **FIG. 11****,** the implant 200 forms an elongated apparatus or article that generally extends along longitudinal axis A. The elongate form of the implant 200 permits it to be tightly compressed and inserted through an intravenous delivery mechanism and in the form shown is referred to as being in the insertion condition.

In operation, a driving implement 228, (e.g., a plastic tube) can be placed over the carrier rod 226 in contact with the second holder 212. The driving implement 228 is slid along or moved toward the first substantially fixed holder 210 (i.e., toward the first end 224 of the carrier rod 226) along the carrier rod 226. Generally, as will be further described, when the driving implement 228 is moved toward the first holder 210 the two holders 210, 212 are driven relatively closer together, and the implant 220 is changed into a deployed condition.

**FIGS. 12-15** show the implant 200 in a perspective view in various conditions of deployment. As can be seen in these figures, moving the two holders 210, 212 together at first tends to cause the inextensible members 202 at the distal end to assume a gently outwardly bowed configuration with respect to the longitudinal axis A of the carrier rod 226. This outwardly bowed configuration pulls the expansible occluding body 214 into a tensioned radial disk shape.

Further moving the driving implement 228 towards the first holder 210 along the carrier rod 226 causes a first portion 230 of the inextensible members 202 located between the first holder 210 and the occluding body 214, to assume a more bowed, almost semi-circular shape where the distal ends 206 of the members 202 attach to the first holder 210 in a nearly perpendicular direction with respect to the longitudinal axis A of the carrier rod 226. A second portion 232 of the inextensible members 202 at the proximal end tends to remain relatively parallel to the longitudinal axis A at first.

The inextensible members 202 are shown in **FIG.13** in a condition in response to yielding to further compression. As shown, the first portions 230 of the members 202 have started to twist or spiral about the carrier rod 226 forming a plurality of generally radial loops (with respect to the axis of the carrier rod). What then happens is that the first portion 230 then passes through a critical point, whereupon each wire in this portion 230 snaps into a generally radially-extending loop, yielding a somewhat concave petal-shaped structure at the distal side of the occluding body 214. Further compressing the second portion 232 likewise results in the formation of similar loops in a petal-shaped structure on the proximal side of the occluding body 214.

**FIGS. 14** and **15** show the implant 200 in a perspective view in a fully deployed condition. Both the first portion 230 and second portion 232 have responded to the compressing motion by twisting away from the axial direction A. Thereby, the first portion 230 of the members have resolved into a first fixation structure 230' adjacent the distal face 216 of the occluding body. Similarly, the second portion 232 of the members 202 have resolved into a second fixation structure 232' adjacent the proximal face 218 of the occluding body 214. The overlapping spiral shape of both the first and second fixation structure 230', 232' is a stable shape, that locks the implant 200 into a stable occluding or deployed condition upon the locking holders 210, 212 being driven together by the driving implement 228, as shown in **FIG.15****.**

**FIG. 16** shows a perspective view of a portion of the implant without the occluding body and members or carrier rod. Thus, the first and second holders or hubs 210, 212 may be seen in more detail. The first and second holders 210, 212 may be adapted to be placed onto the carrier rod (not shown) by hollow central shafts 234, 236. Located about the central shafts 234, 236 are annular portions 238, 240 with a plurality of slots 242 or attachment points sized to fixedly receive the ends of the members 202. The slots 242 may extend to the periphery 244 of the annular portions 238, 240 to allow the members 202 to move from a longitudinal orientation to a radial orientation.

An extending tooth 246 may be formed on one of the first and second holders 210, 212 or in the alternate, the carrier rod (not shown) to engage and form a locking element with a corresponding recess (not shown) formed in one or both of the first and second holders 210, 212. In this manner, the implant 200 becomes locked into a fully deployed or final occluding condition upon being driven together by the driving implement by engagement of the tooth 246 and the recess.

The invention has been carefully described in the above-mentioned examples, and therefore the idea should be clearly understood that the invention is neither limited to the above described and on the drawings shown embodiment, but may be varied within the scope of the claims without departing from the concept of the invention.

## Claims

1. An implant for occluding a passage in a circulatory system, comprising:
a plurality of thin stiff members (202) each having a proximal and a distal end (204, 206);
a first holder (210) to which the distal ends (206) of said members (202) are attached;
a second holder (212) to which the proximal ends (204) of said members (202) are attached;
an expansible occluding body (214) attached to said members (202);
said plurality of members (202) with said occluding body (214) forming an elongated article extending along a longitudinal axis (A) and being adapted for insertion through an intravenous delivery mechanism in an insertion condition;
said members (202) being attached to said first and second holders (210, 212) in a manner to cause said members (202) to execute a twisting motion relative to said axis (A) to yield a plurality of generally radially extending loops, **characterized by**
the expansible occluding body (214) being attached to said members at a point intermediate said first and second holders (210, 212), said occluding body (214) having a distal and proximal face (216, 218);
a carrier rod (226) releasably attached to said first holder (210) and upon which said second holder (212) is slidably received;
a driving implement (228) on said carrier rod (226), said driving implement (228) contacting said second holder (212) and movable toward said first holder (210) along said carrier rod (226);
the radially extending loops combining to form a first fixation structure (230') of generally radially extending loops adjacent said distal face of said occluding body (214), and a second fixation structure (232') of generally radially extending loops adjacent said proximal face of said occluding body (214), wherein said first and second fixation structures (230', 232') are fixed in a final occluding condition upon being driven together by said driving implement (228).

2. The implant of claim 1 further comprising a locking element fixing said first and second fixation structures (230', 232') in a final occluding condition upon being driven together by said driving implement (228).

3. The implant of claim 1 wherein said locking element comprises an extending tooth (246) located on one of said first and second holders (210, 212) which engages with a corresponding recess formed in the other of said first and second holders (210, 212) upon being driven together by said driving implement (228).

4. The implant of claim 1 wherein said members (202) are attached to said first holder (210) at a first angle of inclination relative to said axis (A) and said members (202) are attached to said second holder (212) at a second angle of inclination which is different from said first angle.

5. The implant of claim 4 wherein said twisting motion causes said members (202) attached to said first holder (210) to initially bulge radially outwardly and then snap into an inverted condition having a concave shape for said radially extending loops of said first fixation structure (230'), with the concavity facing said first holder (210).

6. The implant of claim 1 wherein said members (202) are generally inextensible and have a thickened portion where said members (202) attach to said expansible occluding body (214).

7. The implant of claim 1 wherein each said first and second holders (210, 212) include a hollow central shaft portion (234, 236) and an annular hub portion (238, 240) positioned about the central shaft portion (234, 236).

8. The implant of claim 7 wherein each said annular hub portion (210, 212) includes a plurality of slots (242) adapted to respectively receive one of said proximal and distal ends (204, 206) of said members (202).

9. The implant of claim 8 wherein each of said slots (242) extend to an outer periphery of said annular hub portions (238, 240) of said first and second holders (210, 212) to permit said inextensible members (202) to pivot from a first orientation generally parallel to said longitudinal axis (A) to a second orientation generally radial with respect to said longitudinal axis (A).

## Patentansprüche

1. Implantat zum Verschliessen eines Durchgangs in einem Kreislaufsystem, welches umfasst:
eine Vielzahl von dünnen steifen Elementen (202) mit jeweils einem proximalen und einem distalen Ende (204, 206);
einen ersten Halter (210), an welchem die distalen Enden (206) der Elemente (202) angebracht sind;
einen zweiten Halter (212), an welchem die proximalen Enden (204) der Elemente (202) angebracht sind;
einen expandierbaren Verschlusskörper (214), der an den Elementen (202) angebracht ist;
wobei in einem Einführzustand die Vielzahl an Elementen (202) mit dem Verschlusskörper (214) einen langgestreckten Gegenstand bilden, der sich entlang einer longitudinalen Achse (A) erstreckt und zum Einführen durch einen intravenösen Zuführmechanismus geeignet ist;
wobei die Elemente (202) an dem ersten und dem zweiten Halter (210; 212) derart angebracht sind, dass die Elemente (202) veranlasst werden, eine Twistbewegung relativ zur Achse (A) durchzuführen, um eine Vielzahl von sich hauptsächlich radial erstreckenden Schlaufen zu bilden, **gekennzeichnet durch**
ein Anbringen des expandierbaren Verschlusskörpers (214) an den Elementen an einer Stelle zwischen dem ersten und dem zweiten Halter (210, 212), wobei der Verschlusskörper (214) eine distale und eine proximale Fläche (216, 218) aufweist;
einen Trägerstab (226), der lösbar an dem ersten Halter (210) angebracht ist und auf dem der zweite Halter (212) verschiebbar aufgenommen ist;
ein Antriebsgerät (228) auf dem Trägerstab (226), wobei das Antriebsgerät (228) den zweiten Halter (212) kontaktiert und entlang dem Trägerstab (226) in Richtung des ersten Halters (210) beweglich ist;
wobei die sich radial erstreckenden Schlaufen gemeinsam eine erste Fixierungsstruktur (230') aus hauptsächlich sich radial erstreckenden Schlaufen neben der distalen Fläche des Verschlusskörpers (214) und eine zweite Fixierungstruktur (232') aus hauptsächlich sich radial erstreckenden Schlaufen neben der proximalen Fläche des Verschlusskörpers (214) bilden, wobei die erste und die zweite Fixierungstruktur (230', 232') **durch** das aufeinander zu Bewegen mittels dem Antriebsgerät (228) in einem Endverschlusszustand fixiert sind.

2. Implantat nach Anspruch 1, das weiter aufweist, ein Verschlusselement, welches die erste und die zweite Fixierungsstruktur (230',232') in einem Endverschlusszustand fixieren, nachdem Sie mittels dem Antriebsgerät (228) aufeinander zu bewegt sind.

3. Implantat nach Anspruch 1, wobei das Verschlusselement einen abragenden Zahn (246) aufweist, der an dem ersten oder dem zweiten Halter (210, 212) angeordnet ist und der in eine korrespondierende Vertiefung eingreift, die in dem anderen aus erstem oder zweitem Halter (210, 212) ausgebildet ist, wenn diese durch das Antriebsgerät (228) aufeinander zu bewegt sind.

4. Implantat nach Anspruch 1, wobei die Elemente (202) in einem ersten Neigungswinkel relativ zur Achse (A) an dem ersten Halter (210) angeordnet sind und die Elemente (202) an dem zweiten Halter (212) in einen zweiten Neigungswinkel, der sich von dem ersten Winkel unterscheidet, angebracht sind.

5. Implantat nach Anspruch 4, wobei die Twistbewegung die an dem ersten Halter (210) angebrachten Elemente (202) veranlasst, sich anfänglich radial nach aussen zu wölben und dann in einen umgekehrten Zustand umzuschnappen, der eine konkave Form für die sich radial erstreckenden Schlaufen der ersten Fixierungsshuktur (230') aufweist, wobei die Wölbung zum ersten Halter (210) ausgerichtet ist.

6. Implantat nach Anspruch 1, wobei die Elemente (202) im Wesentlichen nicht dehnbar sind und einen verdickten Bereich haben, an dem die Elemente (202) an dem expandierbaren Verschlusskörper (214) angebracht sind.

7. Implantat nach Anspruch 1, wobei der erste und der zweite Halter (210, 212) jeweils einen hohlen zentralen Achsenbereich (234, 236) und einen ringförmigen Buchsenbereich (238, 240), der an dem zentralen Achsenbereich (234, 236) angeordnet ist, umfassen.

8. Implantat nach Anspruch 7, wobei jeder der ringförmigen Buchsenbereiche (210, 212) eine Vielzahl von Schlitzen (242) aufweist, die zur Aufnahme jeweils eines der proximalen und distalen Enden (204, 206) der Elemente (202) geeignet sind.

9. Implantat nach Anspruch 8, wobei sich jeder der Schlitze (242) bis zu einem Aussenumfang des ringförmigen Buchsenbereichs (238, 240) des ersten und zweiten Halters (210, 212) erstreckt, um den nicht dehnbaren Elementen (202) ein Verschwenken von einer ersten im wesentlichen parallel zur longitudinalen Achse (A) verlaufenden Orientierung zu einer zweiten im wesentlichen radial in Bezug zur longitudinalen Achse (A) verlaufenden Orientierung zu ermöglichen.

## Revendications

1. Implant pour l'occlusion d'un passage dans un système circulatoire, comprenant :
une pluralité d'organes rigides minces (202),
ayant chacun une extrémité proximale et une extrémité distale (204, 206) ;
un premier dispositif de retenue (210) auquel les extrémités distales (206) desdits organes (202) sont attachées ;
un deuxième dispositif de retenue (212) auquel les extrémités proximales (204) desdits organes (202) sont attachées ;
un corps d'occlusion extensible (214) attaché auxdits organes (202) ;
ladite pluralité d'organes (202) avec ledit corps d'occlusion (214) formant un article allongé s'étendant le long d'un axe longitudinal (A) et
étant prévus pour être insérés à travers un mécanisme de distribution intraveineux dans un état d'insertion ;
lesdits organes (202) étant attachés auxdits premier et deuxième dispositifs de retenue (210, 212) de manière à exécuter un mouvement de torsion par rapport audit axe (A) pour donner une pluralité de boucles s'étendant généralement radialement,
**caractérisé par**
**le fait que** le corps d'occlusion extensible (214) est attaché auxdits organes en un point entre lesdits premier et deuxième dispositifs de retenue (210, 212), ledit corps d'occlusion (214) ayant une face distale et une face proximale (216, 218) ;
une tige de support (226) attachée de manière détachable audit premier dispositif de retenue (210) et sur laquelle ledit deuxième dispositif de retenue (212) est reçu de manière coulissante ;
un élément d'entraînement (228) sur ladite tige de support (226), ledit élément d'entraînement (228) venant en contact avec ledit deuxième dispositif de retenue (212) et déplaçable vers ledit premier dispositif de retenue (210) le long de ladite tige de support (226) ;
les boucles s'étendant radialement coopérant pour former une première structure de fixation (230') de boucles s'étendant généralement radialement, adjacentes à ladite face distale dudit corps d'occlusion (214), et une deuxième structure de fixation (232') de boucles s'étendant généralement radialement adjacentes à ladite face proximale dudit corps d'occlusion (214), lesdites première et deuxième structures de fixation (230', 232') étant fixées dans un état d'occlusion final lorsqu'elles sont entraînées ensemble par ledit élément d'entraînement (228).

2. Implant selon la revendication 1, comprenant en outre un élément de verrouillage fixant lesdites première et deuxième structures de fixation (230', 232') dans un état d'occlusion final lorsqu'elles sont entraînées ensemble par ledit élément d'entraînement (228).

3. Implant selon la revendication 1, dans lequel ledit élément de verrouillage comprend une dent prolongée (246) située sur l'un desdits premier et deuxième dispositifs de retenue (210, 212), qui s'engage avec un retrait correspondant formé dans l'autre desdits premier et deuxième dispositifs de retenue (210, 212) lorsqu'ils sont entraînés ensemble par ledit élément d'entraînement (228).

4. Implant selon la revendication 1, dans lequel lesdits organes (202) sont attachés audit premier dispositif de retenue (210) suivant un premier angle d'inclinaison par rapport audit axe (A) et lesdits organes (202) sont attachés audit deuxième dispositif de retenue (212) suivant un deuxième angle d'inclinaison qui est différent dudit premier angle.

5. Implant selon la revendication 4, dans lequel ledit mouvement de torsion provoque un bombement initial radialement vers l'extérieur desdits organes (202) fixés audit premier dispositif de retenue (210), puis leur retour rapide dans un état inversé de forme concave pour lesdites boucles s'étendant radialement de ladite première structure de fixation (230'), la concavité faisant face audit premier dispositif de retenue (210).

6. Implant selon la revendication 1, dans lequel lesdits organes (202) ne sont généralement pas extensibles et ont une portion épaissie dans laquelle lesdits organes (202) sont fixés audit corps d'occlusion extensible (214).

7. Implant selon la revendication 1, dans lequel chacun desdits premier et deuxième dispositifs de retenue (210, 212) comporte une portion de tige centrale creuse (234, 236) et une portion de moyeu annulaire (238, 240) positionnée autour de la portion de tige centrale (234, 236).

8. Implant selon la revendication 7, dans lequel chaque dite portion de moyeu annulaire (210, 212) comporte une pluralité de fentes (242) prévues pour recevoir respectivement une desdites extrémités proximale et distale (204, 206) desdits organes (202).

9. Implant selon la revendication 8, dans lequel chacune desdites fentes (242) s'étend vers une périphérie extérieure desdites portions de moyeu annulaire (238, 240) desdits premier et deuxième dispositifs de retenue (210, 212) pour permettre auxdits organes inextensibles (202) de pivoter d'une première orientation généralement parallèle audit axe longitudinal (A) dans une deuxième orientation généralement radiale par rapport audit axe longitudinal (A).
